Europäisches Patentamt

European Patent Office

Office européen des brevets

(11) Veröffentlichungsnummer: **0 255 631**
**A1**

(12) # EUROPÄISCHE PATENTANMELDUNG

(21) Anmeldenummer: **87110331.3**

(22) Anmeldetag: **17.07.87**

(51) Int. Cl.4: **H01M 2/02** , H01M 10/30

(30) Priorität: **01.08.86 DE 8620714 U**

(43) Veröffentlichungstag der Anmeldung:
**10.02.88 Patentblatt 88/06**

(84) Benannte Vertragsstaaten:
**CH DE GB LI**

(71) Anmelder: **C. & E. FEIN GmbH & Co.**
**Leuschnerstrasse 41-47**
**D-7000 Stuttgart 1(DE)**

(72) Erfinder: **Die Erfinder haben auf ihre**
**Nennung verzichtet**

(54) **Sterilisierbare Batterie.**

(57) Die Erfindung betrifft eine Batterie, insbesondere ein Sekundärelement für Antriebseinheiten, die zum Einsatz in der Chirurgie geeignet sind. Es ist Aufgabe der vorliegenden Erfindung, eine Batterie zur Verfügung zu stellen, die im Autoklaven sterilisierbar ist und deren Leistungsfähigkeit durch das Sterilisieren nicht beeinträchtigt wird. Gelöst wird die Aufgabe dadurch, daß die Batterie (1) in einem Gehäuse (2) untergebracht ist, wobei zwischen Batterie (1) und Gehäuse (2) eine Isolierschicht (3) vorgesehen ist. Sinngemäß gilt das gleiche auch zwischen Deckel (5) und Batterie (1). Die Isolierschicht (3) verhindert das Vordringen der erhöhten Temperatur bis zur Batterie (1) während der Aufenthaltszeit im Autoklaven.

Fig.2

## Sterilisierbare Batterie

Die Erfindung betrifft eine Batterie, insbesondere ein Sekundärelement für Antriebseinheiten, die zum Einsatz in der Chirurgie geeignet sind.

Derartige Batterien müssen, um in einem relativ sterilen Operationsraum zum Einsatz zu kommen, in einem Autoklaven sterilisiert werden. Dies könnte erreicht werden, indem man die Batterie bei 134° C mindestens 3 Minuten oder bei 120° C 20 Minuten im Autoklaven läßt.

Bisher bekannte Batterien, die meist zu mehreren in einem Gehäuse zu einem sogenannten Akku-Pack zusammengehalten sind, können auf diese Weise nicht sterilisiert werden, da die Batterien selbst nicht so hochtemperaturbeständig sind und keinen ausreichenden Schutz vor solch hohen Temperaturen haben.

Es ist deshalb Aufgabe der vorliegenden Erfindung, eine Batterie zur Verfügung zu stellen, die im Autoklaven sterilisierbar ist und deren Leistungsfähigkeit durch das Sterilisieren nicht beeinträchtigt wird.

Gelöst wird die Aufgabe dadurch, daß die Batterie in einem Gehäuse untergebracht ist, wobei zwischen Batterie und Gehäuse eine Isolierschicht vorgesehen ist. Sinngemäß gilt das gleiche auch zwischen Deckel und Batterie. Die Isolierschicht verhindert das Vordringen der erhöhten Temperatur bis zur Batterie während der Aufenthaltszeit im Autoklaven.

Die Verwendung von PUR-Schaum als Isolation ermöglicht ein schnelles, preiswertes und exaktes Plazieren der Batterie (oder Batterien) in dem Gehäuse.

Damit das Sterilisieren beliebig oft wiederholt werden kann, ist es sinnvoll das Gehäuse, wie auch den Deckel, aus temperatur-und heißdampfverträglichem Material zu fertigen.

In der Praxis hat sich gezeigt, daß eine Isolierschicht von mindestens 8 mm PUR-Schaum ausreichend Gewähr dafür bietet, daß die Batterie (bzw. die Batterien) durch die Erwärmung nicht in ihrer Leistungsfähigkeit beeinträchtigt wird, insbesondere bei der Verwendung von NiCd-Sekundärzellen.

Wird ein Vakuum zur Isolation zwischen Gehäuse und Batterien verwendet, werden Kriechstrecken vermieden.

Um Leckstellen im Gehäuse oder im Deckel im Bereich der Anschlüsse zu vermeiden, ist es sinnvoll, die Verbindungsleitung zwischen Kontakt und Batterie möglichst lang zu gestalten. Damit wird erreicht, daß die unterschiedlichen Ausdehnungskoeffizient des Gehäuse-(bzw. Deckel-) Materials und der Verbindungsleitung nicht sehr ins Gewicht fällt. Gleichzeitig wird auch der Temperaturübergang von den Kontakten am Gehäuse oder Deckel zur Batterie erschwert. Besondere Montagevorteile bietet eine im Gehäusedeckel eingespritzte Leitung.

Bei Verwendung von Leitgummi oder Kohlenstoff für die Anschlüsse der Batterien wird die Gefahr der Überhitzung beim Sterilisieren reduziert.

Ebenso wie das Gehäuse und der Deckel muß auch der Übergang zwischen Gehäuse und Deckel den hohen Temperaturen und dem heißen Dampf widerstehen. Bei einer Verbindung, die durch Schrauben hergestellt wird, muß deshalb auch eine ensprechende Dichtung verwendet werden oder man stellt die Verbindung durch Kleben oder mittels Ultraschall-Schweißen her.

Ein Ausführungsbeispiel der Erfindung ist anhand von Zeichnungen dargestellt. Es zeigen:

Fig. 1 einen Querschnitt durch eine sterilisierbare Batterieeinheit;

Fig. 2 einen Schnitt nach der Linie II-II in Fig. 1.

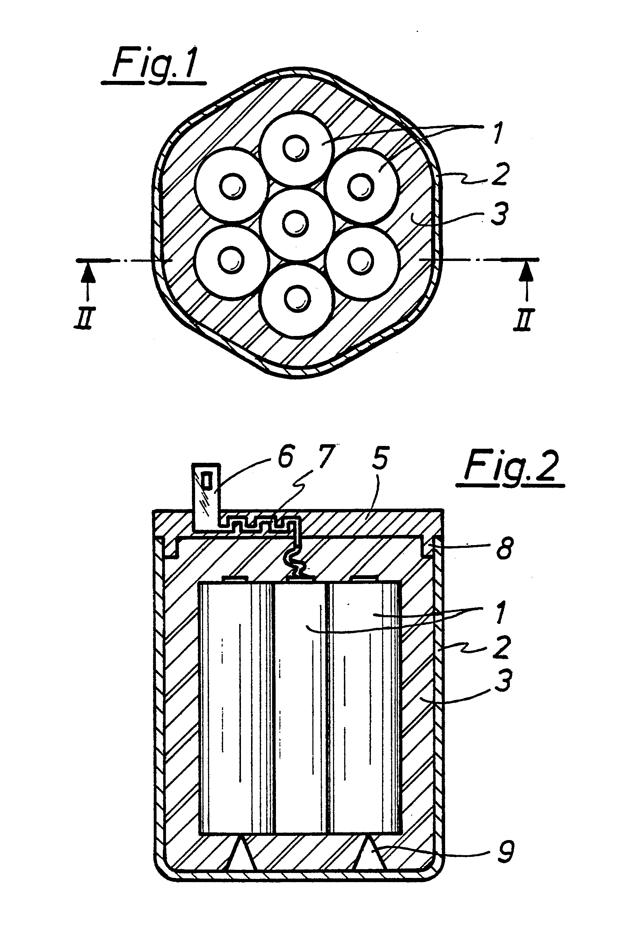

Gemäß Fig. 1 sind die Batterien 1 dicht aneinander mit einem Abstand im Gehäuse 2 angeordnet. Dieser Abstand wird durch eine Isolationsmasse 3 ausgefüllt. Im dargestellten Beispiel ist die Isolationsmasse 3 ein PUR-Schaum, könnte aber von der Isolationswirkung her genausogut Glaswolle oder ein anderes, gut isolierendes Material sein. Als Isolierschicht könnte auch ein Vakuum im Gehäuse 2 dienen.

Der Schnitt nach Fig. 2 zeigt, daß die Batterien 1 auch in einem Abstand zum Gehäuseboden 4 und zum Deckel 5 angeordnet sind und dieser Abstand gleichfalls mit Isoliermasse 3 ausgefüllt ist. Abstandhalter 9 sorgen dafür, daß beim Einsetzen der Baterien 1, z.B. bei der Serienfertigung, diese einen Mindestabstand vom Gehäuse 2 haben. Diese Abstandhalter 9 sind vorzugsweise aus PUR-Schaum oder einem Material mit ähnlichem Temperaturleitwert. Dargestellt sind lediglich Abstandhalter 9 am Boden des Gehäuses 2, es läßt sich aber leicht bewerkstelligen, Abstandhalter an der Wand vorzusehen.

Auf dem Deckel 5 ist ein Kontakt 6 vorgesehen, über den die Batterien 1 ge-und entladen werden können. Über eine in den Deckel 5 eingespritzte Leitung 7 ist der Kontakt 6 mit den Batterien 1 verbunden. Die Leitung 7 ist zur Verlängerung der Wegstrecke mäanderförmig ausgebidlet. Im Ausführungsbeispiel ist der Deckel 5 in das Gehäuse 2 mit seinem Absatz 8 eingeklebt. Wie oben beschrieben, kommen alternativ auch andere Verbindungstechniken in Frage.

**Ansprüche**

1. Batterie, die in einem Gehäuse mit Deckel angeordnet ist, wobei Anschlüsse für die Batterie durch das Gehäuse oder den Deckel geführt sind, **dadurch gekennzeichnet,** daß zwischen dem Gehäuse (2) bzw. Deckel (5) und der Batterie (1) eine Isolierschicht (3) angeordnet ist.

2. Batterie nach Anspruch 1, **dadurch gekennzeichnet,** daß die Isolation (1) aus PUR-Schaum besteht.

3. Batterie nach Anspruch 1, **dadurch gekennzeichnet,** daß das Gehäuse (2) und der Deckel (5) aus temperatur-und heißdampfverträglichem Kunststoff be steht.

4. Batterie nach Anspruch,2 **dadurch gekennzeichnet,** daß die Isolierschicht (3) mindestens 8 mm dick ist.

5. Batterie nach Anspruch 1, **dadurch gekennzeichnet,** daß die Isolierschicht (3) wenigstens teilweise aus einem Vakuum besteht.

6. Batterie nach Anspruch 1, **dadurch gekennzeichnet,** daß die Batterie (1) ein hochtemperaturverträgliches NiCd-Sekundärelement ist.

7. Batterie nach Anspruch 1, **dadurch gekennzeichnet,** daß die Anschlüsse durch das Gehäuse (2) oder den Deckel (5) nicht auf kürzestem Weg, nämlich mäanderförmig, geführt sind.

8. Batterie nach Anspruch 1, **dadurch gekennzeichnet,** daß die Anschlüsse durch das Gehäuse (2) oder den Deckel (5) wenigstens teilweise aus Leitgummi bestehen.

9. Batterie nach Anspruch 1, **dadurch gekennzeichnet,** daß die Anschlüsse durch das Gehäuse (2) oder den Deckel (5) wenigstens teilweise aus Kohlenstoff bestehen.

10. Batterie nach mindestens einem der vorhergehenden Ansprüche, **dadurch gekennzeichnet,** daß die Verbindung zwischen Deckel (5) und Gehäuse (2) mittels einer Dichtung oder durch Kleben bzw. Ultra-schallschweißen luftdicht gestaltet ist.

11. Batterie nach Anspruch 1, **dadurch gekennzeichnet,** daß zwischen Batterie (1) und Gehäuse (2) Abstandhalter (9) vorgesehen sind.

0 255 631

*Fig.1*

*Fig.2*

## EINSCHLÄGIGE DOKUMENTE

EP 87110331.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 514 627 (BRISTOL-MYERS)<br><br>* Anspruch 1 *<br><br>-- | 1,3 | H 01 M 2/02<br>H 01 M 10/30 |
| A | US - A - 3 986 894 (CILIBERTI, JR.)<br><br>* Zusammenfassung *<br><br>-- | 1,3,6 | |
| A | US - A - 4 053 691 (CILIBERTI, JR.)<br><br>* Fig.; Tabelle II *<br><br>---- | 1,2,11 | |

RECHERCHIERTE
SACHGEBIETE (Int C 4

H 01 M 2/

H 01 M 10/

Der vorliegende Recherchenbericht wurde für alle Patentansprüche erstellt.

| Recherchenort | Abschlußdatum der Recherche | Prüfer |
|---|---|---|
| WIEN | 06-11-1987 | LUX |